# EUROPEAN PATENT APPLICATION

(11) **EP 4 458 276 A1**
(43) Date of publication of application: **06.11.2024**
(21) Application number: 23315140.6
(22) Date of filing: 02.05.2023
(51) Int. Cl.: A61B 17/00, A61B 17/04

(54) **CATHETER DEVICES AND METHODS OF CLOSING OPENINGS IN TISSUE**

(71) Applicant: HoliStick Medical, 75013 Paris (FR)
(72) Inventor: The designation of the inventor has not yet been filed
(74) Representative: Hepp Wenger Ryffel AG

(57) **Abstract**

The invention is directed to a catheter device (200) for closing an opening (D) an a tissue (W) which comprising an outer sheath (207) and an inner sheath (208) and an inner shaft (210). The catheter device further comprises a first expandable tissue holder (204, 205) and a piercing head (214). The inner sheath (208) has a longitudinal axis (L) and the inner shaft (210) extends through the inner sheath (207) along the longitudinal axis (L). The first expandable tissue holder (204, 205) is configured to expand into a deployed position. In the deployed position, the first expandable tissue holder (204, 205) defines a first support surface (213) for a tissue (W) to be pierced. The piercing head (214) is movable along the longitudinal axis (L) with respect to the first tissue holder (204, 205). The piercing head (214) further comprises at least two needles (201) pointing in a direction substantially parallel to the longitudinal axis (L) and in a direction of the first support surface (214), and wherein the needles (201) are adapted to carry a pledget (203) which is attached to a suture (202).

## Description

The present invention relates to devices and methods for closing tissue openings according to the preamble of the independent claims.

There are several tissue openings in the human or animal bodies that may need to be closed to treat certain conditions. For example, atrial septal defects such as the patent foramen ovale (PFO) can cause a variety of symptom which can be treated by closing said defect.

Several devices and methods are known in the prior art to close tissue openings.

CN 212394986 discloses a suturing device which is used for minimally invasive surgical suturing.

US 7,846,179 discloses a system for treating a septal defect having a suture-like implantable treatment apparatus, devices for delivering the implantable treatment apparatus and methods for treating a septal defect. The suture-like apparatus is implantable through a septal wall or portion thereof.

US 6,911,034 discloses a suturing apparatus including a needle which is adapted to pierce the inner surface of a biological structure.

US 8,246,636 discloses methods and apparatuses for closing a patent foramen ovale. First and second suture clasp arms are adapted to hold end portions of a suture when in an extended position, and a first suture catch mechanism and a second suture catch mechanism are present.

However, the devices and methods of the prior art are complex to manufacture and use. As a result, the treatment of patients may be long and prone to failure.

Thus, the object of the present invention is to overcome the drawbacks of the prior art, in particular to provide device and methods which are safe and easier to use.

This and other objects are achieved by the medical implant and the methods according to the characterizing portion of the independent claims of the invention.

The present invention is described, in the following, in the context of closure of a PFO in a patient. It will be understood, however, that the devices and methods described herein may be suitable to close other openings in tissue, for example in vessel walls, without departing from the inventive thought.

According to a first aspect, a catheter device is adapted for closing an opening in a tissue. The catheter device comprises an outer sheath, an inner sheath, and at least two piercing elements. Preferably, the catheter device comprises exactly four piercing elements. The inner sheath has a longitudinal axis. The piercing elements are arranged within the inner sheath and have a first section which his reversibly deformable into a bent shape. A second section may have a tip, in particular a needle tip. Additionally or alternatively to a needle tip, the piercing element may be adapted to use radio frequency for piercing the tissue. Each piercing element, in particular the tip, may be adapted to carry a pledget which is attached or attachable to a suture. Additionally or alternatively, the piercing elements may be adapted to carry a suture with no pledget. In this case, the device may comprise a proximal suture catching mechanism. In particular, a mesh may be used which is adapted to pinch a suture. The piercing elements are arrangeable in at least a first and a second position. In the first position, the piercing elements are arranged substantially in parallel to the longitudinal axis such that the tips point in a first direction. In the second position, the second section of the piercing elements is in the bent shape such that the tips point in a second direction which is at least partially opposite the first direction.

The piercing elements may be arranged within the inner shaft in a slideable manner. They may thus be arranged fully within the inner shaft for delivery and moved out of the inner shaft for treatment. A slideable piercing element is advantageous because, it may avoid sharp elements, e.g. needles, from interacting with tissue except when desired to pierce tissue and thus may make the treatment safer. The piercing elements may be stored away within the inner shaft when no treatment is desired.

Within such sharp needles, a suture with a pledget may be arranged, as described above. However, in some cases it is possible that needles are required which are too small for a pledget to fit within the needle. It may thus be advantageous to only arrange a suture within the needle as this allows for smaller structure to be used which may result in a reduced delivery profile.

The catheter device has, in the first position, an elongated shape with a small cross-sectional area which is suitable for minimally invasive treatments. At the same time, the piercing elements may be adapted to be, in the second position, in a variety of different positions relative to one another and to other parts of the device. As such, the device may be easily adapted to perform different treatments and/or to treat openings of different sizes.

In some preferred embodiments, the piercing elements are bendable in a direction away from the longitudinal axis, around a rotation axis which is perpendicular to the longitudinal axis of the inner sheath.

The piercing elements and in particular the tips may be oriented, in the second position, in a direction which is opposite the longitudinal axis of the inner shaft, i.e. in parallel but pointing backwards. However, it will be understood that a strictly opposite orientation is not necessary. In general, the device may be used to extend through the opening, wherein the piercing elements are oriented backwards toward the opening such as to pierce tissue around the opening. As such, any orientation which points in a backwards direction may be suitable, depending on the application, even if the orientation is also partially toward the longitudinal axis, away from the longitudinal axis, and/or skewed with respect to the longitudinal axis.

The piercing elements may be configured to, in the second position, extend away from the longitudinal axis of the shaft evenly distributed, i.e. with constant angles between one another, in a plane perpendicular to the longitudinal axis. However, it is also possible to arrange the piercing elements at any suitable angles.

The tips of the piercing elements may be needle tips and preferably hollow needle tips. The pledgets may be arranged within the needle tips. The suture may be attached to the pledgets and also extend within the needle tip. Alternatively, the sutures may extend through the needle tip outside of the needle.

Pledgets provide a particularly easy and safe way to secure the suture in a tissue. In general, the pledgets may be formed by any element which can be placed in a needle tip and, upon extraction from the needle tip, having a larger cross-section so as to prevent retraction through a tissue hole.

A particularly preferred pledget may be a rod shape which is attached or attachable to a suture in a position substantially in the middle along a rod axis. Thus, the rod may be placed in a needle. If the rod is released from the needle after piercing through a tissue, the rod is prevented from being pulled back through the tissue and thus secures the suture.

The first section may be bendable by any bending mechanism known in the art. In particular, the bending mechanism may comprise an cable associated the first section and which may be pulled. For example, a connection in a handle may be used to pull the cable. The cable may be an inner cable or an outer cable. To bend the bendable arm when needed, the cable may be pulled. When the bendable arm is straight, no tension is put on the cable, when the bendable arm is to bend, then the cable is tensioned and the tension is increased until the bendable arm reaches the desired bend..

The tip of each piercing element may be retracted or retractable into the second section. Preferably, the retraction is reversible.

Retracted or retractable tips, in particular where the tips are needle tips, provide particularly safe treatment. Tips may have sharp and/or pointy elements which may damage surrounding tissue during delivery. Where the tips are retracted or retractable, the piercing elements may additionally be configured to be atraumatic, e.g. with no sharp or pointy elements and/or with soft materials or coatings.

In addition, retracted or retractable tips may provide better control over the treatment in that treatment may only be possible when the tips are not retracted. As such, accidental piercing of tissue, which may occur when the tips are not retracted, is prevented or reduced as the tips may be retracted unless a treatment is to be conducted.

The first section of the piercing element may be formed as a snake chain.

Snake chains provide increase stability and may be adapted to allow a limited bending. As such, a snake chain may be configured to limit the bending to a desired position and orientation which increase control. Snake chain may typically comprise chain elements arranged next to one another, along a longitudinal axis.

The snake chain may provide the shape and freedom for the bendable arm to bend all while providing a stop for the bendable arm not to overbend. The snake chain can be designed with various spacing. In general, higher spacing between elements of a snake chain allows for more bending before reaching a locking effect at a maximum bend.

The second section of the piercing elements may have an opening for a suture. The opening may be arranged such as to be oriented toward the longitudinal axis in the second position of the piercing elements.

Such an opening may allow to direct the suture within the catheter device. For example, the suture may be arranged within the needle tip and exit the needle tip or another portion of the piercing elements so as to avoid the need to arrange the full length of the suture within the piercing elements and/or the device.

In some embodiments, a slot may be used as the opening which allows the release of the suture from the tip, needle tip, and/or piercing elements.

The piercing elements may be rotatable, in particular with respect to one another, about the longitudinal axis of the inner shaft.

As a result, the position of the piercing elements may be adapted during the treatment.

In particular, it may be possible to bend all the piercing elements, from the first position into the second position, substantially along parallel paths and rotate the piercing elements, in the second position rotate the piercing elements with respect to one another into their final position. This approach needs less space to bring the piercing elements from the first to the second position and thus allows treatments in particularly small cavities.

A pledget may be arranged within each needle tip and configured to be ejected through the needle tip. Each pledget may be attached to a suture.

As a result, the sutures are attachable to the tissue in a particularly easy and safe way. When the tissue is pierced and the pledget is ejected on the other side of the tissue or within the tissue, the suture may be secured without the need for any additional measures.

The suture may be connected or connectable to at least a portion, preferably another portion, of the catheter device through the opening.

This may allow for separate and easier control of the suture, i.e. pulling and/or cutting, as such operations may be performed with minimal interaction or handling of the piercing elements and/or the tips.

Furthermore, where the opening is a slot, sutures which are attached to another portion of the catheter device may provide additional safety because the sutures are additionally secured, while still being easily detachable from the piercing elements.

In a second aspect, the invention provides a catheter device for closing an opening in a tissue. The catheter device comprises an outer sheath and an inner sheath, and an inner shaft. The catheter device further comprises a first expandable tissue holder and a piercing head. The inner shaft has a longitudinal axis. The inner shaft extends through the inner sheath along the longitudinal axis. The first expandable tissue holder is configured to expand into a deployed position. Preferably, the first expandable tissue holder is configured such as to expand, for example automatically, when moved out of the inner sheath and/or the outer sheath. In the deployed position, the first expandable tissue holder defines or forms a first support surface for a tissue to be pierced. The piercing head is movable along the longitudinal axis, i.e. in a direction parallel to the longitudinal axis, with respect to the first expandable tissue holder. The piercing head further comprises at least two needles pointing in a direction substantially parallel to the longitudinal axis and in a direction of the first support surface. The needles are adapted to carry a suture, and preferably a suture and a pledget, wherein the suture is attached or attachable to the pledget.

Alternatively to pledgets, the catheter device may be configured to grasp a suture which is delivered through a tissue, on a side of the first tissue support.

In some embodiments, the needles, which form piercing tips, may also be deployable from a delivery position to a deployed position.

Additionally or alternatively to needles, piercing tips suitable to provide radiofrequency piercing may be employed.

To this end, the piercing tips may be connected to an external radio-frequency generator, e.g. positioned bedside, which allows for energy delivery at the piercing tip (e.g. at the tip of the needles). As a result, the tissue may be punctured without mechanical force but rather by burning (pulversing) through the tissue.

The catheter device therefore has a delivery position with a reduced cross-section suitable, for example, for minimally invasive delivery. The catheter device may also provide a particularly safe and easy way of suturing an opening because the piercing elements (e.g. needles or radiofrequency piercing elements) may be deployable into the deployed position, without further manipulation, corresponding to locations to be pierced in the tissue. To this end, the piercing elements may be configured to be elastically deformable into the delivery position and deploy into the deployed position by a spring force once a bias (e.g. from the inner sheath or the outer sheath) is removed. Alternatively, a separate element (for example a cap) may be provided to hold the piercing elements in a delivery position.

Furthermore, the catheter device provides a particularly simple way of attaching a suture to tissue by way of pledgets. It will be understood that pledgets, as described herein, may be used in combination with catheter devices according to any aspect described herein. As such, it will be understood that the use of pledgets may be advantageous, for this and other embodiments, for the reasons described above in the context of the first aspect of the invention.

The pledgets may comprise or consist of a biocompatible material. In particular, the pledgets may comprise or consist of a biocompatible alloy such as stainless steel. Stainless steel is particularly advantageous if high radiopacity is desired, e.g. for imaging of the treated tissue during or after a treatment.

The catheter device according to the second aspect of the invention may have needle adapted to push in a proximal direction or in a distal direction of the catheter device.

It will be understood that the support surface is oriented, with respect to the piercing elements, in a direction in which the piercing elements point such as to pierce the tissue. When used, the support surface may typically be positioned on another side of the tissue to be pierced, compared to the piercing elements. Therefore, the support surface may provide a counterpressure to the piercing elements and thus assist in the piercing.

To this end, the support surface does not need to be a continuous surface or a flat surface. In general, the support surface may be formed by points of contact of expandable arms or struts. The support surface may be curved. The support surface may be configured to exert a force on a tissue, in particular a force sufficient to allow a piercing with a needle from an opposing side of the tissue.

The catheter device may have a second expandable tissue holder which may be configured to expand into a deployed position and forming or defining a second support surface in the deployed position. The first and second tissue holder are arranged such as to support opposing sides of a tissue wall in the deployed positions.

As a result, the tissue to be pierced may be secured more safely and may thus be pierced more accurately and with better control.

The deployed position of the piercing elements may be linked to the deployed position of the first and/or second expandable tissue holder. It also possible, however, that one or both tissue holders and/or the piercing elements are deployable independently of one or both of the respective other elements.

The first and/or the second tissue holder may comprise a plurality of extendable arms.

The piercing head, which may comprise any kind of piercing elements such as needles and/or radiofrequency piercers, may be formed as a distal-most part of the inner sheath. The tissue holder may be arranged on the inner sheath. The piercing elements may point in a proximal direction.

The catheter device may further comprise a piercing head cap which is arranged or arrangeable on the piercing head such as to bias the piercing elements, e.g. the needles, toward the longitudinal axis. When removed, the cap may be configured to release the piercing elements.

A cap provides a simple method to release,piercing elements, such as needles, and may be easily controllable. A cap may be removed by a push or pull force provided through the catheter device.

The piercing head maybe arranged on the inner sheath. The piercing elements, e.g. the needles, may be arranged to point in a distal direction. The first tissue holder may be arranged on the inner shaft.

The needles may comprise a pledget, wherein one pledget may be arranged within each needle and may be configured to be ejected through a needle tip. Each pledget may be attached to a suture.

The pledgets may thus be delivered, for example, onto one side of a tissue after piercing and secure the suture through a pierced hole.

The invention further provides a method of closing an opening in a tissue of a patient. Preferably, a catheter device as described herein is used to perform the method. It will be understood that the catheter devices according to the first and second aspect are particularly suitable, but other catheter devices may of course be employed as well.

The method comprises introducing a catheter device into a patients' body, in particular minimally invasively, and through a tissue opening to be closed. The tissue opening may be an atrial septal defect such as a patent foramen ovale, or an opening in a vessel.

The tissue may be held, at least on a first side of the opening, using a tissue holder.

The tissue is pierced at least twice, i.e. at two different location with respect to the opening, on a second side using at least two piercing elements, for example .needles or radiofrequency piercers. The second side may, in particular, be a distal side of the opening. One pledget may be delivered through each piercing element to the first side. The pledged may in particular be positioned within a needle channel and ejected from the needle channel after piercing. Each pledget may be attached to a suture, wherein each suture may be attached to another portion of the catheter device.

The piercing elements may be pulled back such that the suture passes through the tissue from the first side to the second side.

The sutures may be attached to each other, on the second side, such at to close the opening. A fastener may be used to attach the sutures to one another.

Piercing elements may be deployed into a position pointing at a piercing location, for example by being bent in such an orientation or by being released from a cap, as described herein.

The opening may be a patent foramen ovale.

The catheter device used in the method according to the invention may have exactly four piercing elements, e.g. needles. The step of piercing the tissue may include piercing exactly four areas using the four piercing elements, and delivering four pledgets through the four piercing elements.

A catheter device according to a third aspect of the invention is suitable for suturing an opening in a tissue, for example a patent foramen ovale or any other opening described herein.

The catheter device comprises a main body, a first arm having a first needle hub, a second needle arm having a second needle hub, a first needle, and a second needle. Furthermore, the device comprises a bridge suture and closure suture.

The catheter device has a longitudinal axis and distal end adapted to be delivered through the opening to be closed.

The first arm is arranged at a distal position with respect to the second arm and is pivotable away from the longitudinal axis, preferably in a distal direction. The second arm is pivotable away from the longitudinal axis, preferably in a proximal direction.

A first end of the bridge suture is attached to the first needle hub. A second end of the bridge suture is attached or attachable to, preferably within, the second needle.

The first needle is arranged such as to be ejected from the catheter main body, in particular a bent channel in the catheter main body, into the first needle hub. The closure suture is thereby attached to the first needle hub. The second needle is arranged such as to be ejected into the second needle hub. Thereby, the bridge suture may be attached to the second needle hub.

In general, the catheter device according to the third aspect may be deployed through an opening such that the first and second arms of catheter device are arranged on different sides of the opening. The first arm may be arranged on the distal side of the opening and the second arm on the proximal side of the opening. The first needle, however, may be arranged on the proximal side of the opening and ejected from a channel toward the first needle hub, thereby piercing the tissue. The closure suture may be attached to the first needle and thus attached to the first needle hub and/or the closure suture. The second needle, which may be arranged on the distal side of the opening may be ejected such as to pierce the tissue from the distal side to the proximal side and attach to the second needle hub on the proximal side of the tissue. Pulling on the bridge suture therefore may pull the closure suture through the tissue hole pierced by the second needle and pull along the first needle hub and the closure suture, thereby creating a suture loop formed by the closure suture which extends from a proximal side of the opening, through the pierced hole in the tissue, into a distal side and a back into the proximal side through a second pierced hole in the tissue.

Such a device therefore allows to close an opening using only one closure suture. This facilitates the treatment and the use of the device and minimizes the material which needs to remain in the patient.

The invention further provides a method of closing an opening in a tissue of a patient. The method is advantageously performed using a catheter device according to the third aspect of the invention.

The method comprises arranging a first hub on a second side of a tissue having the opening. The first hub is connected to a first end of a bridge suture.

A closure suture is delivered through the tissue, from a first side to the second side, using a first needle which is pierced through the tissue. The closure suture is attached to the first hub.

A second needle is attached or attachable to a second end of the bridge suture. The second needle is pierced through the tissue to deliver the second end of the bridge suture from the second side to the first side of the tissue. The bridge suture is attached to a second hub arranged on the first side.

The bridge suture is pulled at the second end, such as to pull the closure suture attached to the first end of the bridge suture, through the tissue back to the first side.

The closure suture is secured such as to close the opening.

Preferably, a fastener is used to secure the suture. However, the suture may also be knotted, glued, or attached in any other way known in the art.

Using this method, only one single suture may be used and may be sufficient to close the opening, which results in less material remaining in the patient's heart and thus increased safety.

The first hub may be a first needle hub. The closure suture may be attached or attachable to the first hub via a first needle.

The second hub may be a second needle hub. The bridge suture may be attached or attachable to the second hub via a second needle.

The closure suture may be secured using a fastener.

The invention further provides and implant for closure of an opening. The implant comprises at least one suture which is attached or attachable to a pledget.

Preferably, the implant comprises at least two, in particular exactly four, sutures. Each suture may be attached to a pledget.

Preferably, the implant further comprises a fastener which is configured to secure at least two sutures to one another, in particular on a end of each suture which is generally opposite the pledget.

In general and unless stated otherwise, a distal denominates positions and directions toward an end of the device used for treatment, whereas proximal denominates positions and directions toward the operator/handle.

In the following, the invention is described in detail with reference to the following figures, showing:
- Fig. 1a-1c:: schematically the functioning of a first embodiment of suturing device according to a first aspect.
- Fig. 2:: a second embodiment of a suturing device.
- Fig. 3a-3c:: a third embodiment of a suturing device.
- Fig. 4a-4b:: a perspective and detailed view of handle for a suturing device.
- Fig. 5a-5b:: schematically two alternative functioning principles of a suturing device according to a second aspect.
- Fig. 6:: a first embodiment of a suturing device according to the second aspect.
- Fig. 7:: a detailed view of a first part of the device of Fig. 6.
- Fig. 8:: a detailed view of a second part of the device of Fig. 6.
- Fig. 9:: a cross-sectional view of a handle for a suturing device.
- Figs. 10a-10o:: schematically a treatment using a suturing device according to the second aspect.
- Fig. 11:: a detailed view of a treatment step using the suturing device according to the second aspect.
- Fig. 12:: schematically a suturing device according to a third aspect.
- Fig. 13a-13b:: detailed view of parts of the device of Fig. 12.
- Figs. 14a-14j:: schematically a treatment using a suturing device according to the third aspect.
- Figs. 15a-15c:: schematically the securing of a suture using a first embodiment of a fastener,
- Figs. 16a-16b:: a second embodiment of a fastener.
- Figs. 17a-17b:: schematically sutures secured with fasteners as shown in Figs. 16a and 16b.
- Fig. 18:: a second embodiment of a device according to the second aspect.

In the following, for clarity, certain reference numerals may have been omitted from certain figures, in particular where the same element is present more than once in one. Identical or similar elements are not described repeatedly, for clarity.

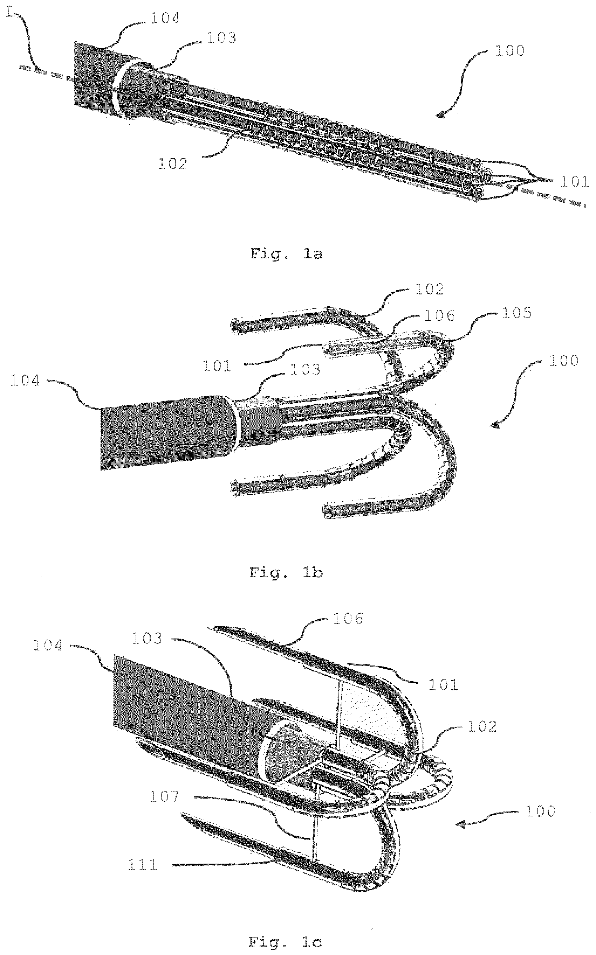

Fig. 1a shows a device 100 according to a first aspect of the invention. The device 100 comprises four piercing elements 101 which are configured as hollow metal rods. The piercing elements 101 comprise a bendable portion 102 which is configured as a snake chain. The piercing elements 101 are arranged within an inner shaft 103 and may be entirely stored within the inner shaft 103 for delivery. Here, the piercing elements 101 extend out of the inner shaft 103 in preparation for a treatment. The inner shaft 103 is arranged within an outer shaft 104. The inner shaft 103, the outer shaft 104 and the piercing elements 101 are arranged, in the shown configuration, in parallel to a longitudinal axis L of the catheter device 100.

Fig. 1b shows the device 100 of Fig. 1a, wherein the piercing elements 101 have been bent by means of the snake chain portion 102. Here, needles 106 are visible inside the piercing elements 101 (shown transparently for clarity) as well as needle pusher elements 105 adapted to push each needle 106 out of the respective piercing element 101.

Fig. 1c shows the device 100 of Figs. 1a and 1b, wherein the needles 106 extend outside of the piercing elements 101. A suture 107 (not shown in Figs. 1a and 1b, for clarity) runs through each needle and to the inner shaft 103. In this configuration, the inner shaft 103 may be pulled back in a proximal direction (i.e. in a direction back into the outer shaft 104), which will also pull the needles 106 in a proximal direction. If the outer shaft extends through an opening (not shown), the needles 106 may thus pierce a tissue and deliver the sutures 107 onto an other side of a tissue. The sutures 107 may be attached using pledgets (not shown) which may be arranged within each needle 107. The needles 106 each comprise a hole 111 through which the sutures 107 extends toward the longitudinal axis L of the catheter device 100. Subsequently, piercing elements 101 may be brought back into the configuration shown in Fig. 1a and retracted into the inner shaft 103, thereby pulling the sutures 107 through the opening for securing.

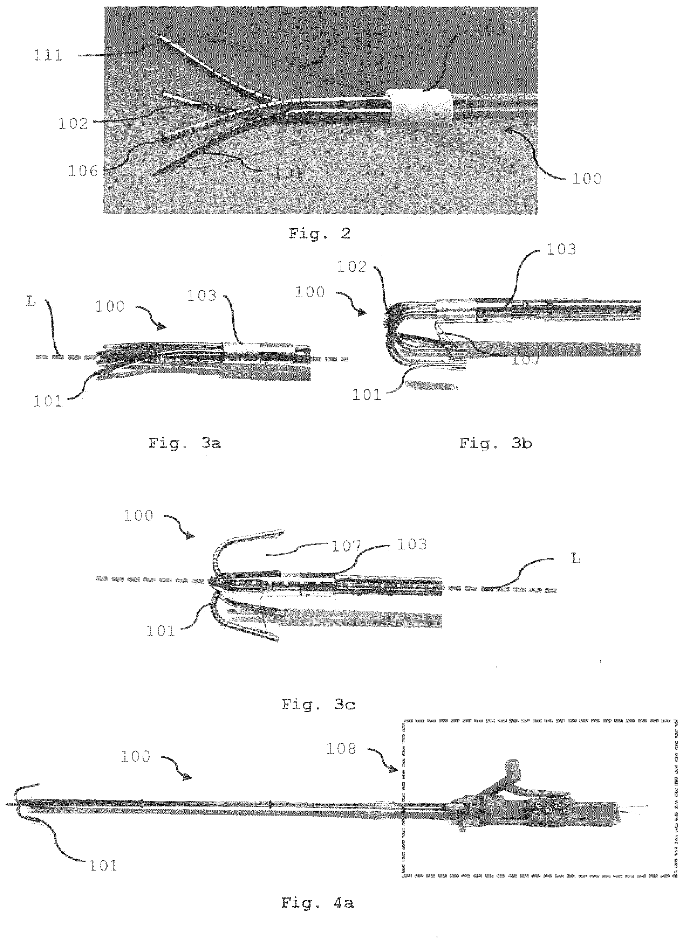

Fig. 2 shows a second embodiment of a device 100 which is similar to the one shown in Figs. 1a-1c. Here, the piercing elements 101, which house retractable needles 106, are fixedly connected to the inner shaft 103. The inner shaft 103 may be arranged slideably in an outer shaft (not shown), however, the piercing elements 101 move together with the inner shaft 103 only. The sutures 107 are attached to the inner shaft 103.

Fig. 3a shows a third embodiment of a device 100 which is similar to the devices shown in Figs. 1a-1c and 2. The configuration of the device 100 shown in Fig. 3a is similar to the one shown in Fig. 1a, wherein the piercing elements 101 extend away, substantially in parallel to the longitudinal axis L.

Fig. 3b shows a configuration of the device 100 wherein the bendable portions of the piercing elements 102 are bent, similar to the configuration shown in Fig. 1b. Here, however, the four piercing 101 are substantially arranged in parallel, i.e. the bendable portions 102 were bent about substantially the same axis. As a result, the piercing elements 101 are all arranged at a similar position with respect to the inner shaft and in particular on the same side of the inner shaft 103.

Fig. 3c shows the configuration for treatment, which is similar to the configuration shown in Fig. 1c. Here, the configuration was obtained by rotation of the piercing elements 101, after bending of the bendable portions 102, around the longitudinal axis L.

Fig. 4a shows a device 100 as shown in Figs. 3a-3c with a handle 108 for controlling the piercing elements 101. It will be understood that a similar handle 108 may also be used with a device as shown in Figs. 1a-1c.

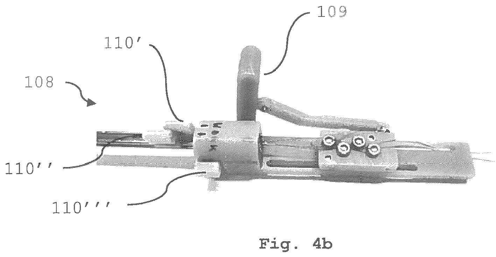

Fig. 4b shows a detailed view of the handle shown in Fig. 4a. The handle 108 comprises an arm pull lever 109 which may be used by a user to bend the piercing elements 101 back as shown e.g. in Figs. 1b and 3b. Furthermore, the handle 108 comprises three axial rotation levers 110', 110'', 110''', each of which control the rotational position of one piercing element 101 with respect to the longitudinal axis L. Here, three axial rotation levers 110', 110", 110‴ are present, meaning that one piercing element 101 cannot directly be rotated. However, other piercing elements 101 are rotated so that any rotational position of the four piercing elements 101 relative to one another can be achieved. It is of course also possible to arrange a fourth axial rotation lever (not shown) to also control the fourth piercing element 101.

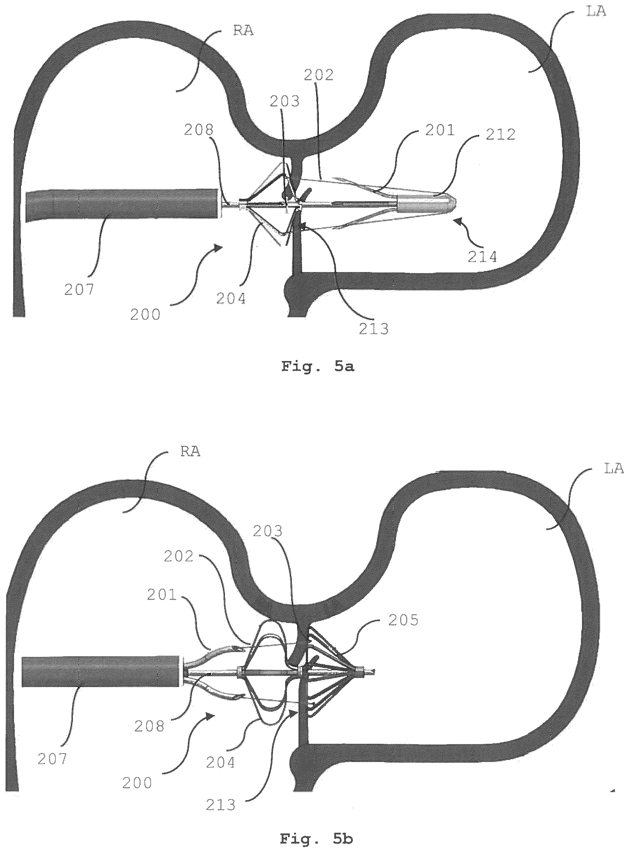

Figs. 5a and 5b show schematically two alternative concepts of a device 200 according to second aspect. The device 200 is used to treat an atrial septal defect and is deployed therethrough, from the right atrium RA into the left atrium. A shaft 207 remains in the right atrium and deployable needles 201 are used to close the defect using multiple sutures 202.

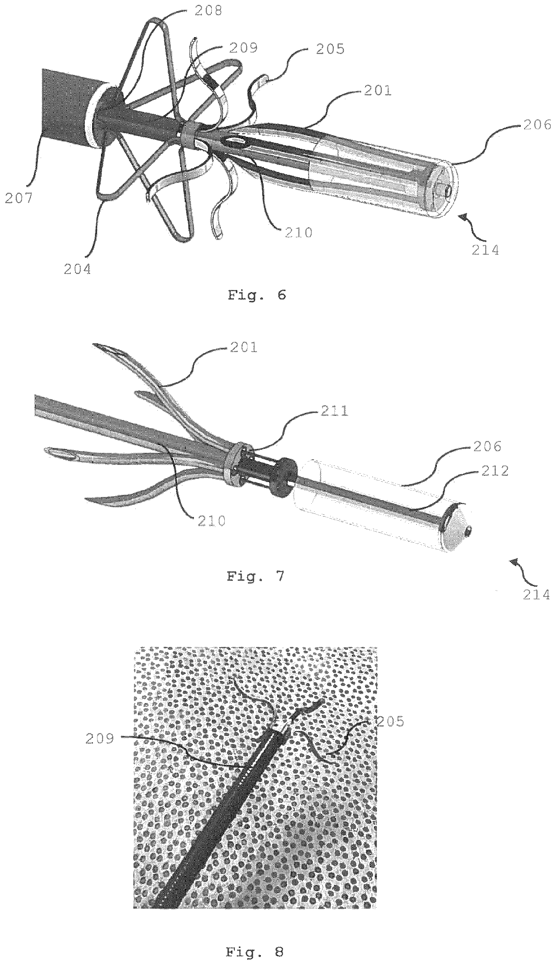

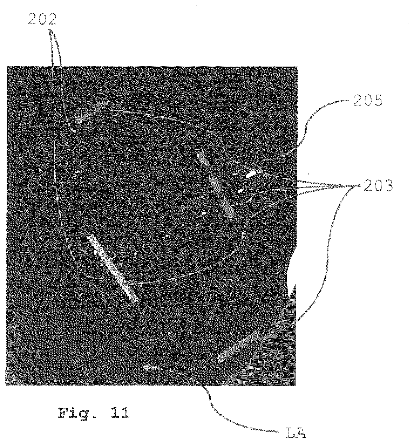

Fig. 5a shows a concept wherein the needles 201 are deployed into the left atrium LA and pierce the tissue from the left atrium LA to the right atrium RA. To this end, the device 200 comprises a expandable support 204 which is configured as a proximal tissue support and which has four arms which extend away, in an arc shape, from an inner shaft element 208. The support 204 is shown in an expanded state in which it is located in the left atrium LA. At a distal position of the support 204, a support surface 213 is formed by the arms which is adapted to provide a force against the tissue acting from the right atrium RA to the left atrium LA. At a distal position of the device 200, a needle head 214 is arranged which comprises four needles 201 and a needle cap 212. The needles 201 are shown in a released state wherein the cap 212 has been removed (see Figs. 6 and 7), causing the needles to be extended away from a central axis. As shown here, the needles 201 have already pierced the tissue and delivered pledgets 203 onto the proximal side of the tissue wall in right atrium RA. The pledgets 203 are each attached to a suture 202 (see Fig. 11) which extends through the tissue and is further attached to the needle cap 212. Therefore, subsequently, the needles 201 may be retracted again by pushing the needle cap 212 over the needles in a proximal direction. The catheter device 200 may be pulled back through the defect, pulling the sutures 202 along, which may then be secured.

Fig. 5b shows an alternative concept of a device 200 which functions substantially similar to the device of Fig. 5b but deploys needles 201 from a proximal direction to pierce in a distal direction, here from right atrium RA into the left atrium LA. To this end, the device 200 comprises a distal tissue support 205 which provides a counter force toward a proximal direction, on a distal side of the tissue to be pierced, in the left atrium LA. The needles 201 are arranged within and deployed from the shaft 207. Along a central axis, the inner shaft 208 extends on which, at a distal end, the tissue support 205 is arranged. In addition, the device 200 shown here has proximal support 204 which provides additional tissue support to more securely pierce the tissue. It will be understood that a proximal anchor 204 is optional in this embodiment. Both tissue supports 204, 205 are configured as expandable wires which may be delivered in an extended elongated shape in which the wires are substantially parallel to the inner shaft 208, and deployed into an arc shape as shown here.

Fig. 6 shows a device 200 which is similar to the device shown in Fig. 5a and is configured to pierce tissue by pulling a piercing head 214 back in a proximal direction toward the shaft 207, when used. The device 200 shown here further comprises a proximal tissue support 205 which is configured as four extending arms which are attached to the proximal tissue support 204. The piercing head 214 is attached on a distal end of a innermost shaft 210which is slidably arranged within a middle shaft 209. The proximal tissue support 204 is slidably arranged on the middle shaft 209 and are formed as an extension of the inner shaft 208. The distal tissue support 205 is attached to the middle shaft 209.

Fig. 7 shows a detailed view of a piercing head 214, for example for use in a device 200 as shown in Figs. 5a and 6. Four needles 201 are arranged on a shaft 210 and configured such as to elastically deform toward the shaft 210 when biased by an external force. A needle holder 211 is attached to a distal end of shaft 210 and holds the needles 201. Through the needles holder 211 and through shaft 210 extends an actuation wire 212 which is attached to needle cap 206 and which is adapted to push the cap in a distal direction and/or to pull the needle cap in distal direction. Therefore, the needles 201 may be retracted, into a delivery position, by pulling the needle cap 212 in a proximal direction over the needles 201. By pushing the needle cap 212 in a distal direction, the needles 201 may be released.

Fig. 8 shows a middle shaft 209 attached to a distal tissue support 205 as isolated elements. The tissue support 205 and the middle shaft 209 may be used, and are substantially similar to the corresponding elements, shown in Fig. 6.

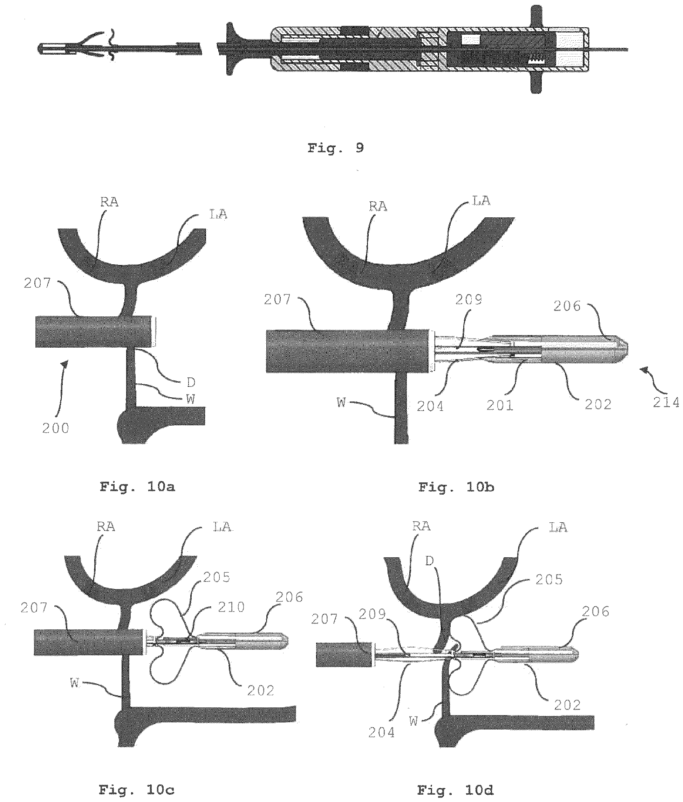

Fig. 9 shows, in an exemplary fashion, a handle which may be used for a device 200 as shown herein. The handle comprises various controls to expand and retract the anchors and the needle assembly and to move the various components axially close or away from one another.

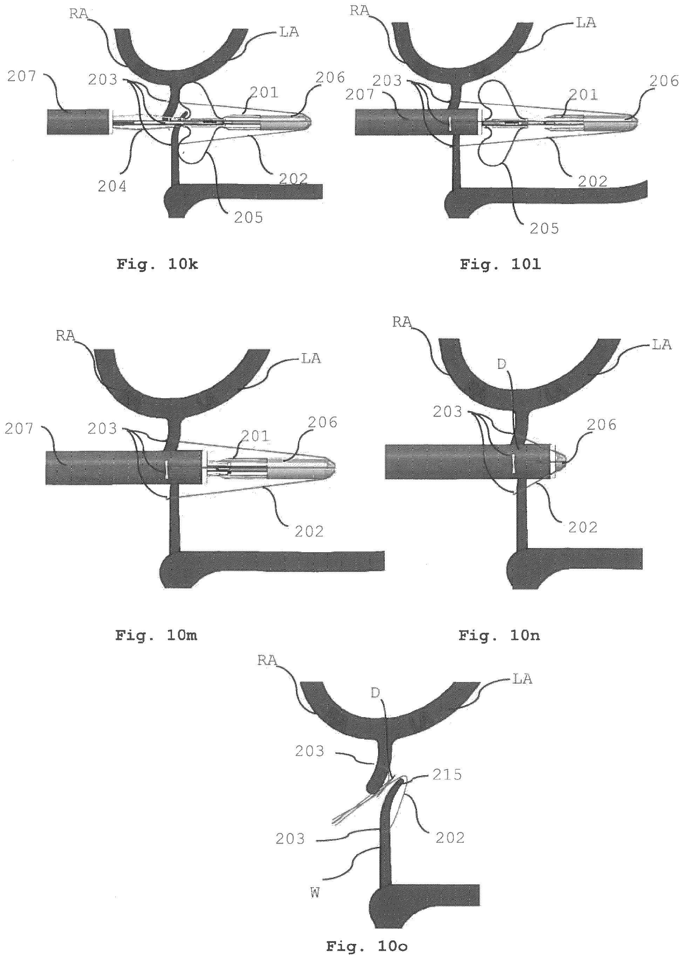

Figs. 10a-10o show a method of treating an opening D in a tissue wall W. The device 200 is similar to the device shown in fig. 6, but it will be understood that other devices, in particular the device of Fig. 5a, may also be employed.

Fig. 10a shows the deployment of a device 200 with a shaft 207 through the opening in the tissue wall W, thereby going from the right atrium RA to the left atrium.

Fig. 10b shows the release of a piercing head 214 from the shaft 207. The piercing head 214 comprises a cap 206 which holds needles 201 in a delivery position. Sutures 202 extend from the needle tips, along an outer area, toward the cap 206. A proximal tissue support 204, slidably arranged on a middle shaft 209, is also in a delivery position.

Fig. 10c shows the deployment of a distal tissue support 205 by expansion of wire arms.

Fig. 10d shows the distal tissue support 205 in fully expanded-configuration. The shaft 207 has been withdrawn from the left atrium LA and the opening D whereby the distal tissue support 205 is pulled to support distal side of wall W in the left atrium LA. The proximal tissue support 204 is in the delivery configuration.

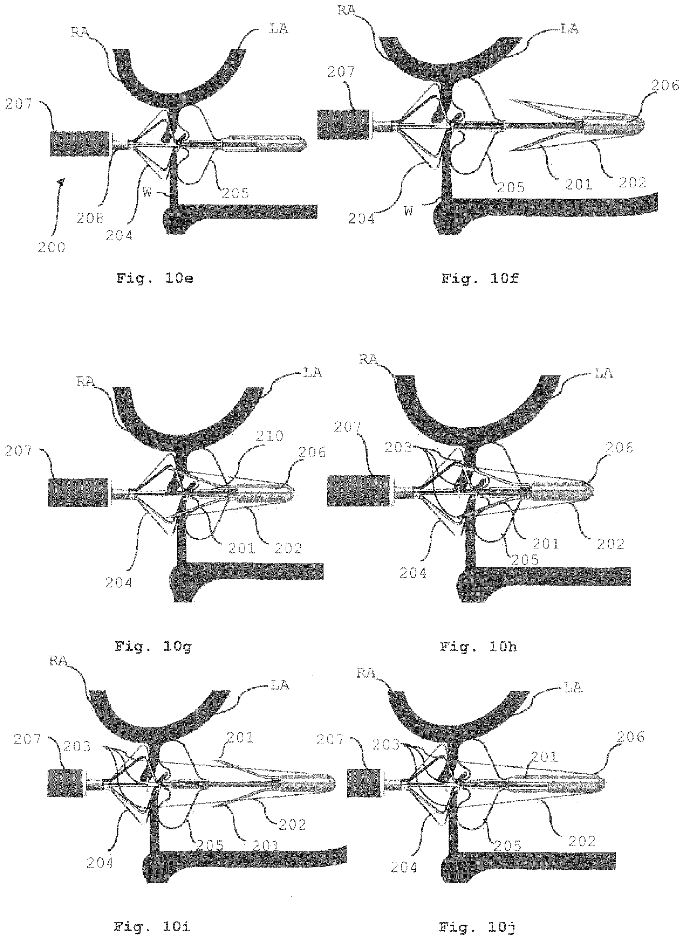

Fig. 10e shows the proximal tissue support 204 in an expanded, deployed position. Shaft 207 is released further and inner shaft 208 is visible. The wall W is pinched between the proximal tissue support 204 and the distal tissue support 205 and is therefore secured and prevented from moving with respect to the device 200.

As shown in Fig. 10f, the needles 201 may be deployed into a piercing position, in which the needles 201 extend away from a central axis of the device 200, by pushing the cap 206 in a distal direction. The sutures 202 extend away from the central axis as well, as the sutures 202 are attached to the cap 206 and extend from within the needles 201.

Fig. 10g shows the needles having pierced through wall W. The sutures 202 are pulled along with the needles 201 and also extend through the tissue wall W.

Fig. 10h shows the release of pledgets 203 from the needles 201. The pledgets 203 are released into the right atrium RA and are configured as medical-grade steel rods with a diameter of 0.6 mm and a length of 6 mm. Each pledget is attached, around a central point along the length, to a suture 202. Accordingly, the pledgets 203 securely attach the sutures to the tissue wall W as the pledgets are prevented from being pulled back through the tissue wall W.

Subsequently, as shown in Fig. 10i, the needles 201 are pulled back and away from the tissue wall W. The pledgets 203 remain in the right atrium RA, on a proximal side of the tissue wall while the sutures 202 extend through the tissue wall to the piercing head 214.

The needles 201 may then be retracted back into a delivery position, as shown in Fig. 10j.

Subsequently, the proximal tissue support 204 is retracted back in to the delivery position, as shown in Fig. 10k.

Then the shaft 207 may moved in a distal direction and into the left atrium LA, as shown in Fig. 10l.

The distal tissue support 205 retracted into the shaft 207 (see Fig. 10m), after which the piercing head 214 may also be retracted into the shaft 207 which is then removed from through the opening D.

The result of the treatment, shown in Fig. 10o, is a suture closure of the opening D. The sutures 202 extend through the tissue wall W and are secured by pledgets on one side of the wall W (here the proximal side), whereas the sutures are attached to one another, here by a knot 215 such as to close the opening D. It will be understood that any type of know of fastener may be used to attach the sutures to one another.

Fig. 11 shows a detailed view of four pledgets 203 with sutures 202 attached thereto. Here, the device (not visible as a whole) is similar to the device shown in Fig. 5b, and a distal tissue support 205 is shown. The pledgets 203 are arranged in the left atrium LA of a patient's heart and delivered by proximal needles pushed through the tissue wall. It will be understood that identical or similar pledgets 203 may be used with any of the devices according to the second aspect, but also with other devices according to the invention.

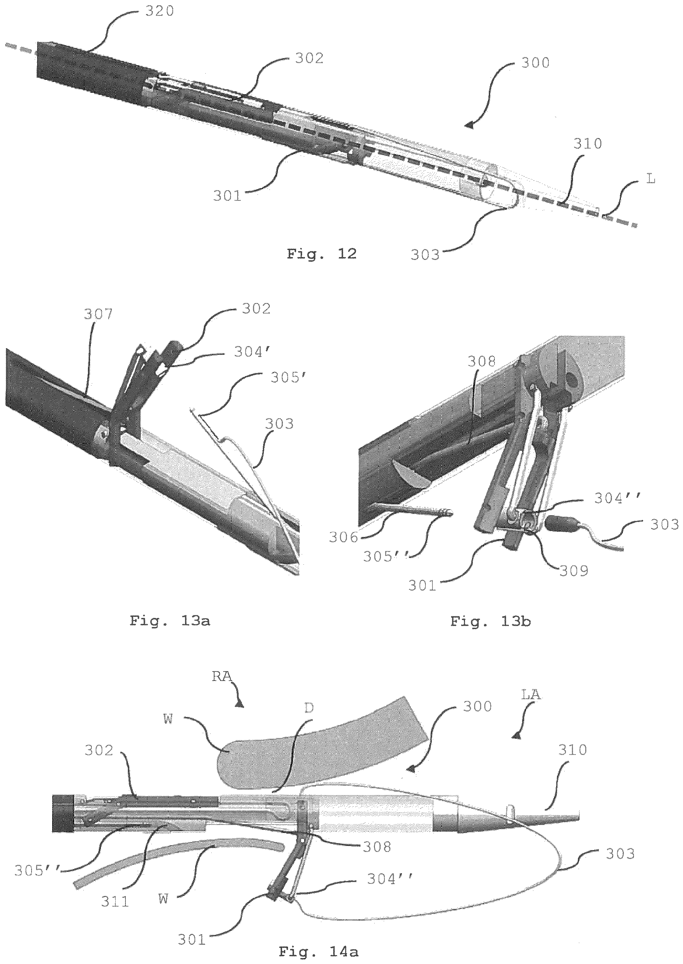

Fig. 12 shows schematically a device 300 according to a third aspect of the invention. The device 300 comprises a catheter main body 320 a soft tip 310 which is atraumatic and prevents tissue damage. The device 300 further has a distal jaw 301 and a proximal jaw 302, and a bridge suture 303. The function of these elements will be explained in detail in the following.

Fig. 13a shows a detailed view of the proximal jaw 302. Here, the proximal jaw 302 is pivoted away from a device main body by means of a pull lever 307 which acts as an opening mechanism.

The proximal jaw 302 opens in a proximal direction. A needle dock 304' is arranged on the proximal jaw and opens, for receiving a needle 305', in a distal direction. The bridge suture 303 is attached with on end to needle 305' and may thus be attached to needle dock 305'.

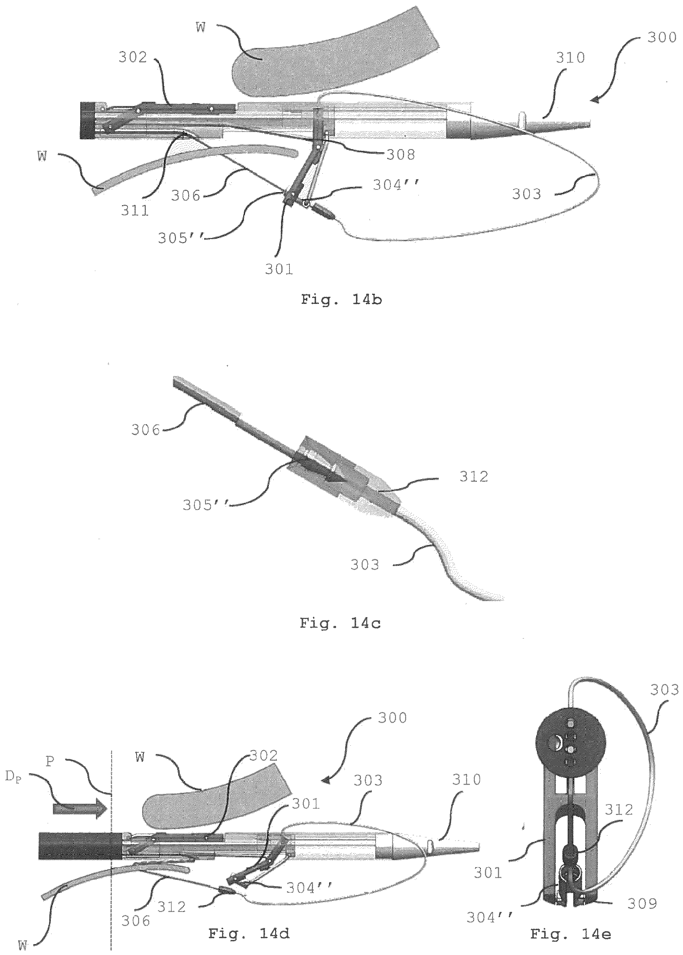

Fig. 13b shows a detailed view of the distal jaw 301. The distal jaw 301 is opened with a push lever 308 and extends away, in the open state, from the main body in a distal direction. The distal jaw 301 comprises a needle dock 304" which may receive a needle 305" on a proximal side. As shown here, needle 305" is attached to closure suture 306. The bridge suture is attachable to the needle dock 304" on a side substantially opposite of where the needle 305'' may be received, i.e. here on the distal side. Needle dock 304" has a slot 309 for release of the bridge suture (see Fig. 14e).

Figs. 14a-14j show schematically a method of treating a patient, by closing a opening D in a tissue using the device of Figs. 13a-13b.

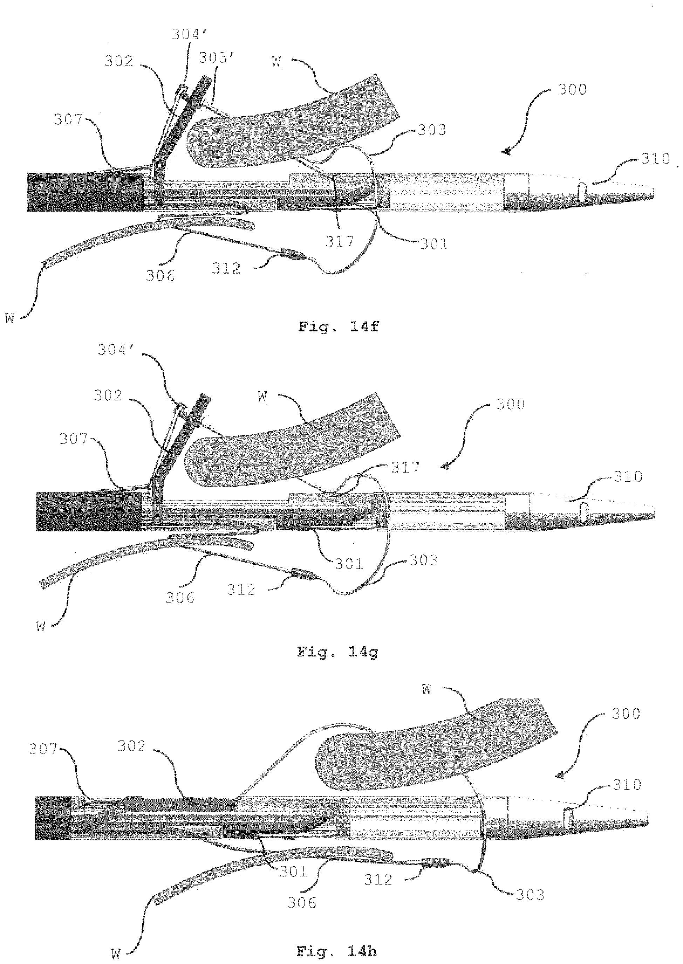

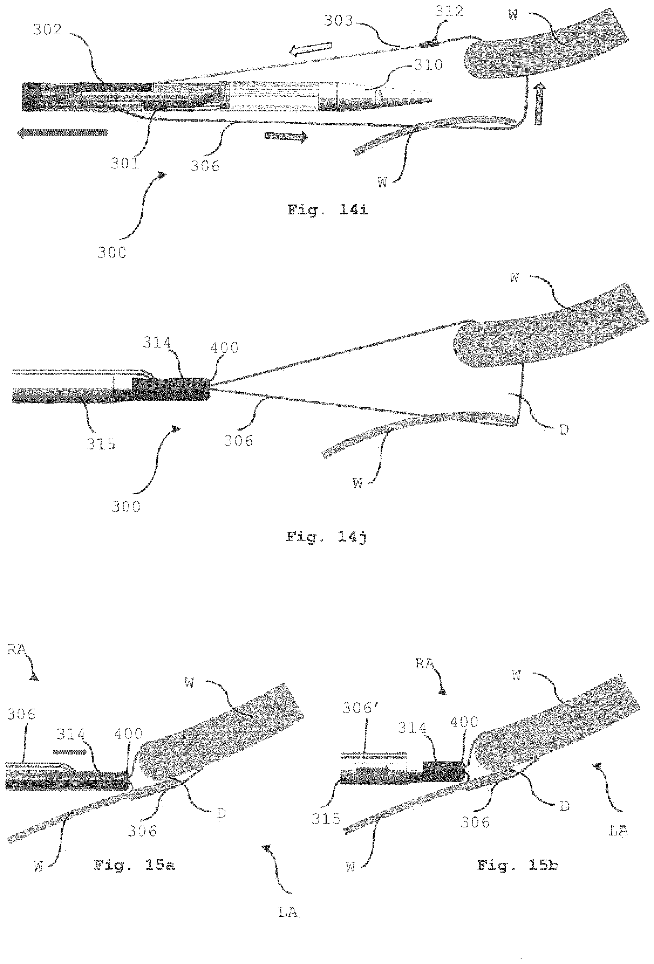

Fig. 14 shows the device 300 being pushed through the opening D in a tissue wall W, from the right atrium RA into the left atrium LA. Here, the proximal jaw 302 is retracted and essentially arranged within the main body of the catheter device 300. The distal jaw 301 is pivoted away from the main body, substantially as shown in Fig. 13b, such as to receive needle 305'' which is arranged within a curved channel 311 in the catheter main body. The bridge suture 303 is attached to the neelde dock 304" of the distal jaw 301. The device 300 is positioned for treatment such that distal jaw 301 is arranged in the left atrium LA of the patient's heart.

Fig. 14b shows a state of the treatment wherein the needle 305'' has been ejected through the curved channel 311. The channel 311 is arranged and oriented such that the needle 305", when ejected, is received in the needle dock 304". By doing so, the needle 305" pierces the tissue wall and pulls suture 306 through the tissue wall W, which is then connected to the bridge suture 303 via the needle dock 304''.

Fig. 14c shows a detailed view of the.bridge suture 303 being connected to closure suture 306 via needle 305'' which is attached to a connector 312. The connector is arrangeable within needle dock 304" (not shown here).

Fig. 14d shows the release of bridge suture 303 from needle dock 304", thereby also releasing closure suture 306. Connector 312 remains attached to the bridge suture 303 and closure suture 306.

Fig. 14e shows a cross-section of the device along plane P of Fig. 14d and viewed in the direction D_{P} indicated in Fig. 14d. Here, slot 309 is visible and through which the bridge suture 303 and the closure suture 306 are released as shown in Fig. 14d. The connector 312, when arranged within the needle dock 304" is prevented from being moved in a lateral direction (i.e. toward the slot 309). However, by pulling the connector 312 out of the needle dock 304" (e.g. by pivoting distal jaw 301) allows the suture, i.e. either the bridge suture 303 or the closure suture 306 depending on the direction of removal of the connector 312, to be removed through slot 309.

As shown in Fig. 14f, the distal jaw 301 may be moved back in a delivery position while the proximal jaw 302 is pivoted away from the catheter main body. The device 300 is positioned such that proximal jaw 302 is positioned in the patient's heart's right atrium RA. A second needle 305' is ejected from a second bent channel 317 and pierces the tissue wall to reach needle dock 304'. The bridge suture 303, which is attached with another end to the needle 305', is attached to the needle dock 304'.

As shown in Fig. 14g, the needle 305' may then be retracted back into channel 317 but leaving bridge suture attached to needle dock 304'.

Proximal jaw 302 may then be retracted, as shown in Fig. 14h. A continuous suture, formed by the bridge suture 303 and the closure suture 306 connected by connector 312, extends from the right atrium RA, through the tissue wall W, into the left atrium LA and back into the right atrium RA through a second piercing in the tissue wall W. Connector 312 is, as shown here, arranged in the left atrium LA, and each of the closure suture 306 and the bridge suture 303 extend through the tissue wall W.

As shown in Fig. 14i, the bridge suture 303 may be pulled and removed such as to pull the closure suture through the pierced hole in which the bridge suture 303 is initially arranged.

As a result, a single suture 306 forms a loop suitable to close opening D in tissue wall W. Here, the device 300 further contains a blade 315 to cut suture 306 as well as a suture clip 400 which is releasable from clip shaft 314, as will be explained in more detail in Figs. 15a-15c. However, the loop formed by suture 306 may be secured by any means known in the art such as knots or glues in order close and secure opening D.

Fig. 15a show a method of secure one or multiple sutures used to close an opening. Here, the method is schematically shown for a treatment performed as shown in Figs. 14a-14j, however, it will be appreciated that similar methods may be employed to secure suture deployed by any other method disclosed herein.

Here, two ends of suture 306 are fastened using a fastener 400 arranged within a clip shaft 314.

As shown in Fig. 15b, a blade 315 may be arranged circumferentially around clip shaft 314 and moved in a distal direction to cut suture 306 so as to remove excess suture 306". Subsequently, suture fastener 400 may be ejected from clip shaft 314.

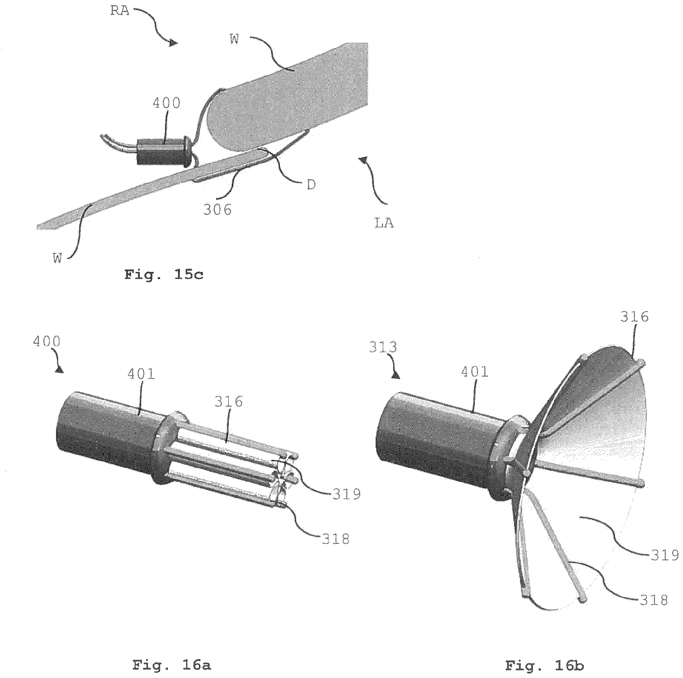

As shown in Fig. 15c, the suture 306 holds opening D closed and is secured by fastener 400. As a result, only a portion of suture 306 is arranged in the left atrium LA. Suture 306 and fastener 400 are in the right atrium RA.

Figs. 16a and 16b show a fastener 400 which may be used as shown in Fig. 15a-15c. The fastener 400 comprises a main body 401 and an umbrella 402 formed by a membrane 404 held by resilient arms 403. Fig. 16a shows the fastener 400 before opening of the umbrella 402 and Fig. 16b shows the fastener after opening of the umbrella 402.

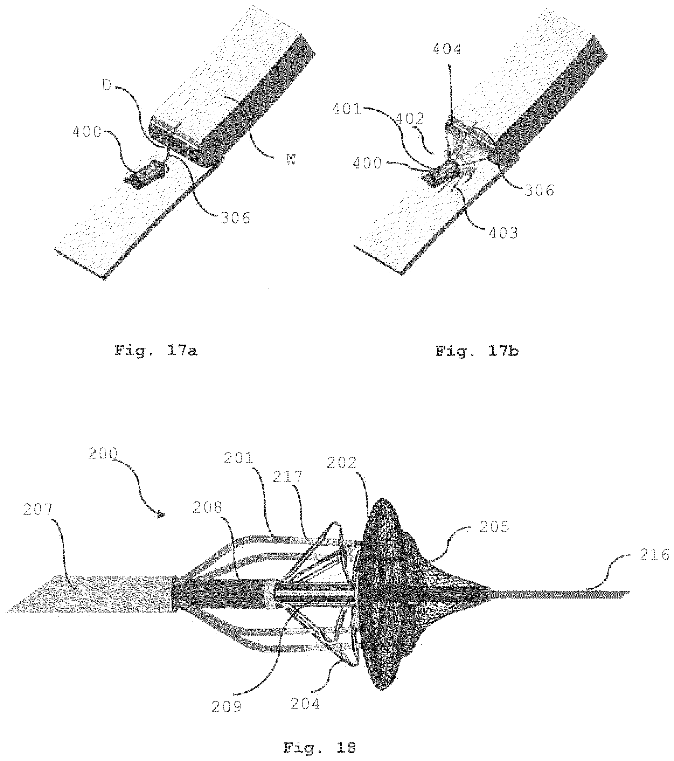

Fig. 17a shows a opening D in a tissue wall W closed with a suture 306 held by a fastener 400 as shown in Fig. 15c in a perspective view.

Fig. 17b a closed opening which is substantially similar as the one shown in Fig. 17a. Here, a fastener 400 as shown in Figs. 16a-16b was used to secure the suture 306. The umbrella 402 provides additional sealing and may thus be advantageous when treating certain openings D that are not fully closed by the suture 306.

Fig. 18 shows a suturing device 200 according to the second aspect. The device is similar, in its functionality, to the device shown in Fig. 5b.

Here, needles 201 extend from within the shaft in a distal direction. In the shown configuration, the needles 201 are deployed from the shaft 207. A distal tissue support 205 is arranged on an inner-most shaft 209 and a proximal support 204 is arranged on an intermediate shaft 208. The fuctionalities of the proximal support 204 and the distal support 205 substantially corresponds to the functionalities described above in the context of Fig. 5b. However, in addition, the distal support 205 is further configured as a mesh. In a fully expanded state, as shown here, the needles 201 can extend through the mesh. A suture. 202 which is ejected from the needles 201 can be temporarily fixed, by being pinched, to the mesh when the proximal support is compressed at least partially. Thus, this present embodiment is particularly suitable when no pledgets (see Fig. 5b) are used to fix the sutures 202 to the tissue. Instead, with the present embodiment, the suture 202 may be delivered through the tissue by piercing and held by the distal support 205 while the needles 201 are retracted back through the tissue. The distal support 205 may then be pulled back through the tissue opening, similar to as shown in Figs. 10m-10n, and bring the sutures 202 back toward the device 200. Another end of the sutures 202 may still be held by the needles 201. As a result, the tissue is held be sutures 202 which may be fastened on one side of the tissue without requiring pledgets.

Here, the device 200 further comprises needle holders 217 attached to the proximal support 204. As a result, the needles 201 are automatically moved in a direction perpendicular to the longitudinal axis, away from the said axis, when the proximal support 204 is deployed. It will be understood that needle holders 217 are not required for this particular embodiment and may be combined with any other embodiment shown herein. The combination shown here is for exemplary purposes only.

Finally, the device 200 shown here further has an inner channel for a guidewire 216.

## Claims

1. A catheter device (200) for closing an opening (D) in a tissue (W), comprising an outer sheath (207) and an inner sheath (208), an inner shaft (210), a first expandable tissue holder (204, 205), and a piercing head (214),
wherein the inner sheath (208) has a longitudinal axis (L) and wherein the inner shaft (210) extends through the inner sheath (207) along the longitudinal axis (L),
wherein the first expandable tissue holder (204, 205) is configured to expand into a deployed position, preferably when moved out of the inner sheath (208) and/or the outer sheath (208), wherein in the deployed position, the first expandable tissue holder (204, 205) defines a first support surface (213) for a tissue (W) to be pierced,
wherein the piercing head (214) is movable along the longitudinal axis (L) with respect to the first tissue holder (204, 205),
**characterized in that** the piercing head (214) further comprises at least two needles (201) pointing in a direction substantially parallel to the longitudinal axis (L) and in a direction of the first support surface (214), and wherein the needles (201) are adapted to carry a suture (202), preferably a suture (202) and a pledget (203) which are attached to one another.

2. The catheter device (200) according to claim 1, further having a second expandable tissue holder (205, 204) configured to expand into a deployed position and defining a second support surface in the deployed position, further wherein the first and second tissue holders (204, 205) are arranged such as to support opposing sides of a tissue wall (W) in the deployed positions.

3. The catheter device (200) according to any one of claims 1 or 2, wherein the first and/or the second tissue holder (204, 205) comprises a plurality of extendable arms.

4. The catheter device (200) according to any one of claims 1 to 3, wherein the piercing head (214) is formed as a distal-most part of the inner shaft (210), preferably wherein the first expandable tissue holder (204) is arranged on the inner sheath, and wherein the needles are arranged such as to point in a proximal direction.

5. The catheter device (200) according to claim 4, further comprising a piercing head cap (206) arranged or arrangeable on the piercing head (214) such as to bias the needles (201) toward the longitudinal axis (L), and configured to release the needles (201) when removed.

6. The catheter device (200) according to any one of claims 1 to 3, wherein the piercing head (214) is preferably arranged on the inner sheath (208), and the needles (201) are arranged to point in a distal direction, preferably wherein the first tissue holder (205) is arranged on the inner shaft (210).

7. The catheter device (200) according to any one of claims 1 to 6, further comprising a pledget (203) arranged within each needle (201) and configured to be ejected through a needle tip, wherein each pledget (203) is attached to a suture (202).

8. A catheter device (100) for closing an opening (D) in a tissue (W), comprising a sheath, preferably an outer sheath (104) and an inner sheath (103), and at least two piercing elements (101), preferably exactly four piercing elements (101),
wherein the sheath (103, 104) has a longitudinal axis (L), wherein the piercing elements (101) are arranged within the sheath (103, 104) and have a first section which is reversibly deformable into a bent shape (102), and a second section having a tip (106), preferably a needle tip (106), further wherein each piercing element (101), preferably the needle tip (106), is adapted to carry a suture (107), preferably a suture (107 and a pledget (203) which are attached or attachable to one another,
wherein the piercing elements (101) are arrangeable in at least a first and a second position,
wherein in the first position, the piercing elements (101) are arranged substantially in parallel to the longitudinal axis (L) such that the tips (106) point in a first direction,
and wherein in the second position, the second section of the piercing elements is in the bent shape such that the tips (106) points in a second direction which is at least partially opposite the first direction.

9. The catheter device (100) according to claim 8, wherein the tip (106) of each piercing element (101) is, preferably reversibly, retracted or retractable into the second section.

10. The catheter device (100) according to any one of claims 8 or 9, wherein the first section of the piercing elements (101) is formed as a snake chain.

11. The catheter device (100) of any one of claims 8 to 10, wherein the second section of the piercing elements (101) has an opening for a suture (107), preferably wherein the opening is arranged such as to be oriented toward the longitudinal axis (L) in the second position of the piercing elements (101).

12. The catheter device (100) according to any one of the preceding claims, wherein the piercing elements (101) are rotatable with respect to one another about the longitudinal axis (L).

13. The catheter device (100) according to any one of claims 8 to 12, further comprising a pledget (203) arranged within each tip (106) and configured to be ejected through the tip (106), wherein each pledget (203) is attached to a suture (107) .

14. The catheter device (100) according to claims 11 and 13, wherein the suture (107) is connected or connectable to at least a portion of the catheter device (200) through the opening (111).

15. A method of closing an opening (D) in a tissue (W) of a patient, preferably using a catheter device (100, 200) according to any one of the preceding claims, comprising
introducing a catheter device (100, 200) into a patient's body and through a tissue opening (D) to be closed,
preferably holding the tissue (W) on at least a first side of the opening (D) using a tissue holder (204, 205),
piercing the tissue (W) at least twice, on a second side, using at least two needles (106, 201), preferably wherein the first side is a proximal side and the second side is a distal side of the opening (D), and delivering one pledget (203) through each needle (106, 201) to the first side, wherein the each pledget (203) is attached to a suture (107, 202),
pulling the needle back such that each suture (107, 202) passes through the tissue (W) from the first side to the second side,
attaching the sutures (107, 202) on the second side such as to close the opening (D).

16. The method according to claim 15, wherein the opening (D) is a patent foramen ovale.

17. The method according to any one of claims 15 or 16, wherein the catheter device (100, 200) comprises exactly four needles (106, 201), wherein the step of piercing the tissue (W) includes piercing exactly four times using the four needles (106, 201), and delivering four pledgets (203) are delivered through the four needles (106, 201).

18. A catheter device (300) for suturing an opening (D) in a tissue (W), comprising a catheter main body (320), a first arm (301) having a first needle hub (304"), a second arm (302) having a second needle hub (304'), a first needle (305''), a second needle (305'), a bridge suture (303), and a closure suture (306),
the catheter device (300) having a longitudinal axis (L) and a distal end adapted to be delivered through the opening (D) to be closed,
the first arm (301) being arranged at a distal position with respect to the second arm (302) and being pivotable away from the longitudinal axis, preferably in a distal direction, and the second arm (302) being pivotable away from the longitudinal axis (L), preferably in a proximal direction,
wherein a first end of the bridge suture (303) is attached to the first needle hub (304'') and a second end of the bridge suture (303) is attached or attachable to, preferably within, the second needle (305'),
wherein a first needle (305") is arranged such as to be ejected from the catheter main body (320) into the first needle hub (304"), thereby attaching the closure suture (306) to the first needle hub (304"),
and wherein the second needle (305') is arranged such as to be ejected into the second needle hub (304').

19. A method of closing an opening (D) in a tissue (W) of a patient, preferably using a catheter device (300) according to claim 18, comprising
arranging a first hub (304'') on a second side of a tissue (W) with an opening (D), wherein the first hub (304'') is connected to a first end of a bridge suture (303),
delivering a closure suture (306) through the tissue (W), from a first side to the second side, using a first needle (305") which is pierced through the tissue (W), and attaching the closure suture (306) to the first hub (304"),
using a second needle (305') which is attached to a second end of the bridge suture (303) to pierce through the tissue (W) and deliver the second end of the bridge suture (303) from the second side to the first side, and attaching the bridge suture (303) to a second hub (304') arranged on the first side,
pulling the bridge suture (303) at the second end, such as to pull the closure suture (306) attached to the first end of the bridge suture (303), through the tissue (W) back to the first side,
securing the closure suture (306) such as to close the opening.

20. The method of claim 19, wherein the first hub (304'') is a needle hub, and the closure suture (306) is attached to the first hub (304") via a first needle (305").

21. The method of any one of claims 19 or 20, wherein the second hub (304') is a needle hub, and the bridge suture (303) is attached to the second hub (304') via a second needle (305').

22. The method of any one of claims 19 to 21, wherein the closure suture (306) is secured using a fastener (400).
